# EUROPÄISCHE PATENTSCHRIFT

(11) **EP 0 980 376 B1**
(45) Veröffentlichungstag und Bekanntmachung des Hinweises auf die Patenterteilung: **18.06.2003**
(21) Anmeldenummer: 98921472.1
(22) Anmeldetag: 20.04.1998
(51) Int. Cl.: C07H 13/06

(54) **VERFAHREN ZUR HERSTELLUNG VON KOHLENHYDRATPARTIALESTERN**
METHOD FOR PRODUCING CARBOHYDRATE PARTIAL ESTERS
PROCEDE DE PREPARATION D'ESTERS PARTIELS D'HYDRATE DE CARBONE

(30) Priorität: 28.04.1997 DE 19717968
(43) Veröffentlichungstag der Anmeldung: 23.02.2000
(73) Patentinhaber: Cognis Deutschland GmbH & Co. KG, 40589 Düsseldorf (DE)
(72) Erfinder: CLAVERIE, Valérie, F-31100 Toulouse (FR); CECUTTI, Christine, F-31240 Saint Jean (FR); MOULOUNGUI, Zephirin, F-31500 Toulouse (FR); GASET, Antoine, F-31000 Toulouse (FR); LE HEN FERRENBACH, Catherine, F-77100 Meaux (FR)
(86) Internationale Anmeldenummer: EP9802322
(87) Internationale Veröffentlichungsnummer: WO98049175

(56) Entgegenhaltungen:
- EP-A- 0 132 941
- EP-A- 0 315 265
- EP-A- 0 644 211
- WO-A-92/11270
- DE-A- 4 131 505
- GB-A- 2 256 869

## Beschreibung

### Gebiet der Erfindung

Die Erfindung betrifft ein verbessertes Verfahren zur Herstellung von Kohlenhydratpartialestern durch Umesterung von Glykosen mit Fettsäureestem in Gegenwart von Emulgatoren und basischen Katalysatoren sowie die Verwendung der nach dem Verfahren erhältlichen Stoffe für eine Reihe von Anwendungen.

### Stand der Technik

Kohlenhydratester, die auch häufig vereinfachend als "Zuckerester" bezeichnet werden, stellen Ester von Mono- oder Oligosacchariden, im weiteren Sinne auch von Zuckeralkoholen, mit organischen oder anorganischen Säuren dar. Kohlenhydratester besitzen ausgeprägte grenzflächenaktive Eigenschaften, weshalb man sie heute als eigenständige Verbindungsklasse (sogenannte Zuckertenside) betrachtet. Wegen ihrer guten dermatologischen und toxikologischen Verträglichkeit dienen Kohlenhydratester überwiegend als Emulgatoren zur Herstellung von Lebensmitteln und Kosmetika. Saccharosepolyester mit 6 bis 8 Fettsäureresten könnten als vom Organismus nicht verwertbares Fettsubstitut in der Ernährung übergewichtiger Personen dienen u. sollen außerdem LDL-Cholesterin im Darm binden. Zu ihrer Herstellung geht man üblicherweise von Glykosen aus, die in Gegenwart von alkalischen Katalysatoren und gegebenenfalls Emulgatoren mit Fettsäuremethylestem einer Umesterung unterworfen werden; ein solches Verfahren wird beispielsweise in der deutschen Patentanmeldung **DE-A1 4131505** (Henkel) beschrieben. Als Emulgatoren für die Herstellung der Kohlenhydratester werden in der Regel Seifen eingesetzt, wie dies beispielsweise in der britischen Offenlegungsschrift **GB-A 22256869** offenbart wird.

Von Nachteil bei den bekannten Verfahren des Stands der Technik ist jedoch, daß zur Einstellung insbesondere höherer Veresterungsgrade lange Reaktionszeiten erforderlich sind, was die Produkte wegen der erheblichen Kesselbelegungszeiten verteuert. Infolge der längeren Temperaturbelastung sind die Produkte auch häufig verfärbt. Schließlich müssen häufig Lösungsmittel mitverwendet werden, was nicht wegen der anschließenden Abtrennung zu einer weiteren Verteuerung der Produkte führt, sondern auch im Hinblick auf einen Einsatz beispielsweise im Lebensmittelbereich häufig unerwünscht ist.

Demzufolge hat die Aufgabe der Erfindung darin bestanden, ein verbessertes Verfahren zur Herstellung von Kohlenhydratpartialestem zur Verfügung zu stellen, das frei von den geschilderten Nachteilen ist.

### Beschreibung der Erfindung

Gegenstand der Erfindung ist ein Verfahren zur Herstellung von Kohlenhydratpartialestern mit einem Veresterungsgrad im Bereich von 1 bis 6 durch alkalikatalysierte Umesterung in Gegenwart von Emulgatoren, bei dem man
(a) zur Bildung eines katalytisch aktiven Systems Alkalicarbonate mit Fettsäureniedrigalkylestern der Formel **(I)** behandelt,

   **R**^{**1**}**CO-OR**^{**2**} **(I)**

   in der R¹CO für einen linearen oder verzweigten, gesättigten oder ungesättigten Acylrest mit 6 bis 22 Kohlenstoffatomen und R² für einen linearen oder verzweigten Alkylrest mit 1 bis 5 Kohlenstoffatomen steht, und
(b) zur Umesterung die so erhaltene Mischung unter starkem Rühren mit einer Mischung aus (b1) Glykosen mit 5 bis 12 Kohlenstoffatomen und (b2) Kohlenhydratpartialestern als Emulgatoren behandelt, so daß eine Emulsion/Dispersion resultiert, in der die Teilchen einen mittleren Durchmesser von 10 bis 60 µm aufweisen.

Im ersten Schritt des Verfahrens entsteht ein Überzug von Fettsäureniedrigalkylester auf dem Alkalicarbonat, wodurch die Acylgruppe aktiviert wird. Im zweiten Schritt wird der aktivierte Katalysator mit einer Mischung aus einer Glykose und einem Kohlenhydratpartialester in Kontakt gebracht, wobei letzterer als Emulgator wirkt. Im ersten Schritt der Umesterung findet eine Übertragung von Acylgruppen auf den Emulgator statt, der in der weiteren Folge der Reaktion selbst als Acylierungsmittel fungiert und Acylgruppen auf die Glykose überträgt, die ihrerseits damit in einen Kohlenhydratpartialester überführt wird. Die Reaktion findet in Abwesenheit von Lösungsmitteln statt, was aus ökonomischer Sicht, aber auch im Hinblick auf eine Verwendung im Lebensmittel- oder Kosmetiksektor von erheblichem Vorteil ist. Ein weiterer unerwarteter Vorteil des Verfahrens besteht darin, daß man durch die effektive Art der Umesterung im Vergleich zum Stand der Technik gleiche Veresterungsgrade in sehr viel kürzeren Reaktionszeiten erzielt.

### Katalysatoren

Als Katalysatoren kommen Alkalicarbonate, vorzugsweise Natrium- und/oder Kaliumcarbonat in Frage, die in Mengen von 5 bis 50, vorzugsweise 10 bis 20 und insbesondere 10 bis 15 Gew.-% - bezogen auf die Fettsäureniedrigalkylester - eingesetzt werden können.

### Fettsäureniedrigalkylester

Typische Beispiele für geeignete Acylierungsmittel sind die Ester von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Methanol, Ethanol, Propanol, Isopropylalkohol, n-Butanol, i-Butanol, tert.Butanol, n-Pentanol und Isopentanol. Vorzugsweise werden Methyl- und/oder Ethylester von Fettsäuren mit 12 bis 18 Kohlenstoffatomen eingesetzt, wobei das molare Einsatzverhältnis Ester : Glykose in Abhängigkeit von dem gewünschten Veresterungsgrad 1 : 3 bis 3 : 1, vorzugsweise 1 : 2 bis 2 : 1 betragen kann.

### Herstellung des Katalysatorsystems

Zur Herstellung des Katalysatorsystems, d.h. zur Aktivierung der Fettsäurealkylester als Acylierungsmittel, werden Ester und Alkalicarbonat unter starkem Rühren vermischt. Hierbei hat es sich als vorteilhaft erwiesen, die Aktivierung bei Temperaturen im Bereich von 40 bis 120, vorzugsweise 80 bis 100°C durchzuführen. Die Alkalicarbonatmenge liegt dabei vorzugsweise im Bereich von 5 bis 50 Gew.-% bezogen auf den Alkylester. Hierbei findet eine Chemisorption des Esters auf der Oberfläche des Carbonats statt.

### Glykosen

Unter den Glykosen werden die auch als Kohlenhydrate bezeichneten Polyhydroxyaldehyde (Aldosen) und Polyhydroxyketone (Ketosen) sowie höhermolekulare Verbindungen zusammengefaßt, die sich durch Hydrolyse in solche Stoffe überführen lassen. Im Sinne der Erfindung können als Glykosen sowohl die monomeren Polyhydroxyaldehyde oder Polyhydroxyketone (Monosaccharide) oder ihre Dimeren bis Decameren (Disaccharide, Trisaccharide, Oligosaccharide) eingesetzt werden. Als Monosaccharide (auch "einfache Zucker" genannt) kommen beispielsweise Biosen, Triosen, Tetraosen, Pentosen, Hexosen, Heptosen etc. in Frage. Typische Beispiele für Aldopentosen sind D-Ribose, D-Xylose und L-Arabinose. Zu den wichtigsten Aldohexosen gehören D-Glucose, D-Mannose und D-Galactose; bei den Ketohexosen sind D-Fructose und Sorbose zu nennen. Die 6-Desoxyzucker L-Fucose und L-Rhamnose sind ebenfalls weit verbreitete Hexosen und kommen als Ausgangsstoffe ebenfalls in Frage. Die einfachsten Oligosaccharide, die als Ausgangsstoffe geeignet sind, stellen die Disaccharide dar. Vorzugsweise werden Saccharose (Rohrzucker, Rübenzucker), Lactose (Milchzucker) und/oder Maltose (Malzzucker) eingesetzt. Der Einsatz von Mono- und/oder Disacchariden ist im Sinne des Verfahrens bevorzugt; insbesondere werden Saccharose oder Glucose eingesetzt.

### Emulgatoren

Im Sinne der Erfindung hat es sich als besonders vorteilhaft erwiesen, als Emulgatoren Kohlenhydratpartialester einzusetzen, die mit den Zielprodukten identisch sind. Besonders bevorzugt ist der Einsatz von Saccharosepartialestern mit einem Veresterungsgrad im Bereich von 1 bis 3. Als Co-Emulgatoren kommen weiterhin z.B. nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-Anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. Die Herstellung von C_{8/18}-Alkylmono- und -oligoglycosiden erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glykosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Im Sinne der Erfindung hat es sich jedoch als besonders vorteilhaft erwiesen, als Emulgatoren Kohlenhydratpartialester einzusetzen, die mit den Zielprodukten identisch sind. Die Kohlenhydratester-Emulgatoren und die Glykosen werden im molaren Verhältnis von 1 : 6 bis 6 : 1, vorzugsweise 1 : 5 bis 5 : 1 eingesetzt; der Anteil der Co-Emulgatoren kann im Bereich von 5 bis 50, vorzugsweise 10 bis 25 Gew.-% - bezogen auf die Emulgatoren - liegen.

### Umesterung

Die Umesterung wird dergestalt durchgeführt, daß aus unter starkem Rühren eine Emulsion/Dispersion hergestellt wird, in welcher das Katalysatorsystem, die Glykose und der Kohlenhydratester sowie gegebenenfalls weitere Emulgatoren enthalten sind. Die Emulsion/Dispersion weist eine mittlere Teilchengröße von 10 bis 60, vorzugsweise 20 bis 40 µm auf. Die Umesterung findet in dieser Emulsion/Dispersion vorzugsweise bei Temperaturen im Bereich von 100 bis 160, insbesondere 120 bis 140°C und bei vermindertem Druck im Bereich von 1 bis 10, vorzugsweise 2 bis 8 mbar statt, wobei die Reaktionszeiten im Bereich von 1 bis 4 h liegen. Freiwerdender Alkohol kann im Vakuum leicht abdestilliert werden. Auf diese Weise werden Kohlenhydratpartialester hergestellt, die vorzugsweise einen Veresterungsgrad von 1 bis 6 und vorzugsweise 1 bis 3 besitzen.

### Gewerbliche Anwendbarkeit

Die nach dem erfindungsgemäßen Verfahren erhältlichen Kohlenhydratester weisen ausgezeichnete grenzflächenaktive Eigenschaften auf und können beispielsweise als Emulgatoren für die Herstellung von Lebensmitteln (Brot, Backwaren, Speiseeis etc.) und kosmetischen Präparaten dienen, in denen sie in Mengen von 0,1 bis 10, vorzugsweise 1 bis 5 Gew.-% enthalten sein können. In besonderer Weise geeignet sind die Kohlenhydratester als Emulgatoren zur Herstellung von Polyacryl- bzw. Polymethacryäureverbindungen, die als Superadsorber beispielsweise für Babywindeln dienen können. Da die Emulgatoren im Produkt verbleiben, kommen hier nicht nur ihre ausgezeichneten anwendungstechnischen Eigenschaften, sondern auch ihre besondere Hautverträglichkeit zum Tragen. Ein weiterer Gegenstand der Erfindung betrifft daher die Verwendung der nach dem erfindungsgemäßen Verfahren erhartlichen Kohlenhydratester als Emulgatoren zur Herstellung von Lebensmitteln, kosmetischen Produkten und Superadsorbern.

### Beispiele

**Beispiel 1.** In einem 500-ml-Dreihalskolben mit schnellaufendem Rührer, Rückflußkühler und Tropftrichter wurden 47,5 g (0,2 mol) gehärteter Palmfettsäuremethylester vorgelegt und auf 65°C erwärmt. Bei einer Rührergeschwindigkeit von 1000 UpM wurden 5 g (0,035) Kaliumcarbonat, entsprechend 12 Gew.-% bezogen auf dem Methylester zugegeben. Anschließend wurde die entstandene Dispersion über den Tropftrichter portionsweise mit 10 g (0,02 Mol) eines Ester der Saccharose mit Palmfettsäure (Veresterungsgrad ca. 2) versetzt. Nach 15 min Rühren wurde die Rührgeschwindigkeit auf 2000 UpM gesteigert und 37,5 g (0,1 mol) Saccharose zugegeben (Molverhältnis Methylester : Saccharose = 2). Anschließend wurde die resultierende Emulsion/Dispersion bei einer Rührergeschwindigkeit von 300 UpM, einer Temperatur von 120°C und einem verminderten Druck von 7 mbar weitere 4 h gerührt. Es resultierte ein Saccharoseester mit einem Gehalt von 15 Gew.-% Monoester, 25 Gew.-% Diester und 60 Gew.-% höheren Homologen - bezogen auf die Menge Kohlenhydratester. Der Gehalt an nichtabregiertem Ester in der Reaktionsmischung lag unter 1 Gew.-%, der Gehalt an freier Saccharose bei 6 Gew.-%.

**Beispiel 2.** Beispiel 1 wurde mit 56,8 g (0,2 mol) Talgfettsäuremethylester, 10 g Kaliumcarbonat (entsprechend 18 Gew.-% bezogen auf Methylester), 15 g (0,03 mol) Saccharoseester und 51,3 g (0,15 mol) Saccharose (Molverhältnis Methylester : Saccharose = 1,3) wiederholt. Es resultierte ein Saccharoseester mit einem Gehalt von 11 Gew.-% Monoester, 29 Gew.-% Diester und 60 Gew.-% höheren Homologen - bezogen auf die Menge Kohlenhydratester. Der Gehalt an nichtabregiertem Ester in der Reaktionsmischung lag unter 1 Gew.-%, der Gehalt an freier Saccharose bei 7 Gew.-%.

## Patentansprüche

1. Verfahren zur Herstellung von Kohlenhydratpartialestem mit einem Veresterungsgrad im Bereich von 1 bis 6 durch alkalikatalysierte Umesterung in Gegenwart von Emulgatoren, **dadurch gekennzeichnet, daß** man
(a) zur Bildung eines katalytisch aktiven Systems Alkalicarbonate mit Fettsäureniedrigalkylestern der Formel **(I)** behandelt,
**R**^{**1**}**CO-OR**^{**2**} **(I)**
in der R¹CO für einen linearen oder verzweigten, gesättigten oder ungesättigten Acylrest mit 6 bis 22 Kohlenstoffatomen und R² für einen linearen oder verzweigten Alkylrest mit 1 bis 5 Kohlenstoffatomen steht, und
(b) zur Umesterung die so erhaltene Mischung unter starkem Rühren mit einer Mischung aus (b1) Glykosen mit 5 bis 12 Kohlenstoffatomen und (b2) Kohlenhydratpartialestern als Emulgatoren behandelt, so daß eine Emulsion/Dispersion resultiert , in der die Teilchen einen mittleren Durchmesser von 10 bis 60 µm aufweisen.

2. Verfahren nach Anspruch 1, **dadurch gekennzeichnet, daß** man als Alkalicarbonate Natriumund/oder Kaliumcarbonat einsetzt.

3. Verfahren nach den Ansprüchen 1 und 2, **dadurch gekennzeichnet, daß** man Ester der Formel **(I)** einsetzt, in der R² für einen Methyl- und/oder Ethylrest steht.

4. Verfahren nach den Ansprüchen 1 bis 3, **dadurch gekennzeichnet, daß** man das katalytisch aktive System gemäß (a) bei Temperaturen von 40 bis 120°C herstellt.

5. Verfahren nach den Ansprüchen 1 bis 4, **dadurch gekennzeichnet, daß** man als Glykosen Monound/oder Disaccharide einsetzt.

6. Verfahren nach den Ansprüchen 1 bis 5, **dadurch gekennzeichnet, daß** man als Emulgatoren Kohlenhydratpartialester einsetzt, die mit den Zielprodukten identisch sind.

7. Verfahren nach den Ansprüchen 1 bis 6, **dadurch gekennzeichnet, daß** man als Emulgatoren Saccharosepartialester mit einem Veresterungsgrad im Bereich von 1 bis 3 einsetzt.

8. Verfahren nach den Ansprüchen 1 bis 7, **dadurch gekennzeichnet, daß** man die Umesterung gemäß (b) bei Temperaturen im Bereich von 100 bis 160°C und bei vermindertem Druck im Bereich von 1 bis 10 mbar durchführt.

9. Verwendung von Kohlenhydratpartialestern nach dem Verfahren nach den Ansprüchen 1 bis 8 als Emulgatoren zur Herstellung von Lebensmitteln und kosmetischen Zubereitungen.

10. Verwendung von Kohlenhydratpartialestern nach dem Verfahren nach den Ansprüchen 1 bis 8 als Emulgatoren zur Herstellung von Superadsorbern auf Poly(meth)acrylatbasis.

## Claims

1. A process for the production of carbohydrate partial esters with a degree of esterification of 1 to 6 by alkali-catalyzed transesterification in the presence of emulsifiers, **characterized in that**
(a) to form a catalytically active system, alkali metal carbonates are treated with fatty acid lower alkyl esters corresponding to formula **(I)**:
**R**^{**1**}**CO-OR**^{**2**} **(I)**
in which R¹CO is a linear or branched, saturated or unsaturated acyl group containing 6 to 22 carbon atoms and R² is a linear or branched alkyl group containing 1 to 5 carbon atoms, and
(b) for transesterification, the resulting mixture is treated while stirring vigorously with a mixture of (b1) glycoses containing 5 to 12 carbon atoms and (b2) carbohydrate partial esters as emulsifiers, so that an emulsion/dispersion in which the particles have a mean diameter of 10 to 60 µm is obtained.

2. A process as claimed in claim 1, **characterized in that** sodium and/or potassium carbonate is/are used as the alkali metal carbonate(s).

3. A process as claimed in claims 1 and 2, **characterized in that** esters corresponding to formula (I) in which R² is a methyl and/or ethyl group are used.

4. A process as claimed in claims 1 to 3, **characterized in that** the catalytically active system according to (a) is prepared at temperatures of 40 to 120°C.

5. A process as claimed in claims 1 to 4, **characterized in that** mono- and/or disaccharides is/are used as the glycoses.

6. A process as claimed in claims 1 to 5, **characterized in that** carbohydrate partial esters identical with the target products are used as emulsifiers.

7. A process as claimed in claims 1 to 6, **characterized in that** sucrose partial esters with a degree of esterification of 1 to 3 are used as emulsifiers.

8. A process as claimed in claims 1 to 7, **characterized in that** the transesterification according to (b) is carried out at temperatures of 100 to 160°C and under a reduced pressure of 1 to 10 mbar.

9. The use of carbohydrate partial esters produced by the process claimed in claims 1 to 8 as emulsifiers for the production of foods and cosmetic preparations.

10. The use of carbohydrate partial esters produced by the process claimed in claims 1 to 8 as emulsifiers for the production of poly(meth)acrylate-based superadsorbers.

## Revendications

1. Procédé de préparation d'esters partiels d'hydrate de carbone ayant un degré d'estérification dans la zone de 1 à 6, par transestérification catalysée par un alcali, en présence d'agents émulsionnants,
**caractérisé en ce qu'**
a) on traite en vue de la formation d'un système actif catalytiquement, des carbonates de métal alcalin avec des esters d'alkyle inférieur d'acide gras, de formule (I)
R¹CO-OR² (I)
dans laquelle R¹CO représente un reste acyle saturé on non saturé, linéaire ou ramifié, ayant de 6 à 22 atomes de carbone, et R² représente un reste alkyle linéaire ou ramifié, ayant de 1 à 5 atomes de carbone, et
b) on traite en vue de la transestérification le mélange ainsi obtenu sous forte agitation avec un mélange à base de (b1) des glycoses ayant de 5 à 12 atomes de carbone et (b2) des esters partiels d'hydrate de carbone comme agents émulsionnants de façon qu'il en résulte une émulsion/dispersion, dans laquelle les particules possèdent un diamètre moyen de 10 à 60 µm.

2. Procédé selon la revendication 1,
**caractérisé en ce qu'**
on met en oeuvre comme carbonates de métal alcalin, du carbonate de sodium et/ou de potassium.

3. Procédé selon les revendications 1 et 2,
**caractérisé en ce qu'**
on met en oeuvre des esters de formule (I) dans laquelle R² représente un reste méthyle et/ou éthyle.

4. Procédé selon les revendications 1 à 3,
**caractérisé en ce qu'**
on prépare le système catalytique conformément à (a), à des températures de 40 à 120°C.

5. Procédé selon les revendications 1 à 4,
**caractérisé en ce qu'**
on met en oeuvre comme glycoses, les mono- et/ou des disaccharides.

6. Procédé selon les revendications 1 à 5,
**caractérisé en ce qu'**
on met en oeuvre comme agents émulsionnants des esters partiels d'hydrate de carbone qui sont identiques aux produits cibles.

7. Procédé selon les revendications 1 à 6,
**caractérisé en ce qu'**
on met en oeuvre comme agents émulsionnants un ester partiel de saccharose ayant un degré d'estérification dans la zone de 1 à 3.

8. Procédé selon les revendications 1 à 7,
**caractérisé en ce qu'**
on effectue la transestérification conformément à b) à des températures dans la zone de 100 à 160°C et à une pression réduite dans la zone de 1 à 10 mbar.

9. Utilisation des esters partiels d'hydrate de carbone selon le procédé selon les revendications 1 à 8, comme agents émulsionnants en vue de la préparation de produits alimentaires et de préparations cosmétiques.

10. Utilisation d'esters partiels d'hydrate de carbone selon le procédé selon les revendications 1 à 8, comme agents émulsionnants en vue de la préparation de superabsorbants à base de poly(méth) acrylates.
